# EUROPEAN PATENT SPECIFICATION

(11) **EP 2 992 087 B1**
(45) Date of publication and mention of the grant of the patent: **30.12.2020**
(21) Application number: 14792047.4
(22) Date of filing: 02.05.2014
(51) Int. Cl.: C12N 5/071, C12N 5/079, G01N 33/50

(54) **IDENTIFICATION AND ISOLATION OF HUMAN CORNEAL ENDOTHELIAL CELLS (HCECs)**
IDENTIFIZIERUNG UND ISOLIERUNG MENSCHLICHER HORNHAUTENDOTHELZELLEN
IDENTIFICATION ET ISOLEMENT DE CELLULES ENDOTHÉLIALES DE LA CORNÉE HUMAINE (HCEC)

(30) Priority: 03.05.2013 US 201361819146 P
(43) Date of publication of application: 09.03.2016
(73) Proprietor: Emmetrope Ophthalmics LLC, Menlo Park, CA 94025 (US); Goldberg, Jeffrey L., Menlo Park, CA 94025 (US); Kunzevitzky, Noelia J., Menlo Park, CA 94025 (US)
(72) Inventor: GOLDBERG, Jeffrey, L., Menlo Park, CA 94025 (US); KUNZEVITZKY, Noelia, J., Menlo Park, CA 94025 (US)
(74) Representative: Hocking, Adrian Niall
(86) International application number: PCT/US2014/036616
(87) International publication number: WO 2014/179716

(56) References cited:
- EP-A1- 1 130 032
- EP-A1- 3 029 140
- WO-A1-2005/038015
- WO-A1-2013/012087
- US-A1- 2011 236 347
- LILI TAKÁCS ET AL: "Stem cells of the adult cornea: From cytometric markers to therapeutic applications", CYTOMETRY. PART A, vol. 75A, no. 1, 1 January 2009 (2009-01-01), pages 54-66, XP055294759, US ISSN: 1552-4922, DOI: 10.1002/cyto.a.20671
- ALENA BARTAKOVA ET AL: "Novel Identity and Functional Markers for Human Corneal Endothelial Cells", INVESTIGATIVE OPTHALMOLOGY & VISUAL SCIENCE, vol. 57, no. 6, 19 May 2016 (2016-05-19), page 2749, XP055294684, US ISSN: 1552-5783, DOI: 10.1167/iovs.15-18826
- SMITH ET AL.: 'Expression and regulation of activated leukocyte cell adhesion molecule in human retinal vascular endothelial cells' EXP EYE RES. vol. 104, 24 August 2012, pages 89 - 93, XP028956978
- YOSHIDA ET AL.: 'Tight junction transmembrane protein claudin subtype expression and distribution in human corneal and conjunctival epithelium' INVEST OPHTHALMOL VIS SCI. vol. 50, 30 December 2008, pages 2103 - 2108, XP055290338
- SUNDBERG M ET AL: "CD marker expression profiles of human embryonic stem cells and their neural derivatives, determined using flow-cytometric analysis, reveal a novel CD marker for exclusion of pluripotent stem cells", STEM CELL RESEARCH, ELSEVIER, NL, vol. 2, no. 2, 1 March 2009 (2009-03-01), pages 113-124, XP025991511, ISSN: 1873-5061, DOI: 10.1016/J.SCR.2008.08.001 [retrieved on 2008-09-16]

## Description

### BACKGROUND OF THE INVENTION.

When the innermost layer of the cornea, the endothelium, is damaged, for example from trauma (e.g., from cataract surgery), disease or dystrophy, the cornea swells with fluid (edema) and loses its optical clarity. Patients consequently suffer from vision loss and pain, and their only option to treat advanced disease is with corneal transplant surgery (also known as penetrating keratoplasty, PK) or Descemet's stripping endothelial keratoplasty (DSAEK), both technically difficult procedures that are very invasive to the patient and have significant limitations, such as the number of donor corneas available.

Recent studies have proposed the use of human corneal endothelial cells (HCECs) obtained from cadaveric donors to replace the damaged cells. See, e.g., Joyce and Zhu, Cornea. 2004 Nov;23(8 Suppl):S8-S19; Engelmann, et al., Exper. Eye Res., vol. 78, no. 3, pp. 573-578, 2004. A potential advantage to such an approach could be the expansion of HCECs ex vivo before implantation into patients, thereby overcoming the limited tissue availability. HCECs can be expanded in defined tissue culture media for at least 5 passages, greatly expanding the number of cells derived from a single donor.

One of the main problems with such a technique is that the lack of defined surface markers specific for HCECs makes it difficult to confirm the identity of HCECs after several passages, or to select HCECs away from contaminating cells, or to identify the subset of HCECs that are likely to have the highest clinical efficacy from among the full population of HCECs, as current identification criteria are limited to cell morphology and the expression of functional genes, such as ATP1A1 (see, e.g., Kaye and Tice, Invest Ophthalmol. 1966; 522- 32; Leuenberger and Novikoff, J Cell Biol. 1974; 60721- 731; McCartney et al., Curr Eye Res. 1987; 61479-1486) or the tight-junction marker zonula occludens -1(ZO-1) (see, e.g., Petroll et al., Curr Eye Res. 1999 Jan; 18(1):10-9), neither of which are specific to HCECs. It is also difficult to isolate HCECs from contaminant fibroblasts in culture, from neighboring cells in whole corneas, or from residual corneas from DSAEK.

In this regard, the current isolation method for obtaining HCECs from intact corneas comprises a peel-off step, where the endothelium and its basement membrane (Descemet's membrane) are peeled off the stroma and collected. See, e.g., Ko-Hua Chen et al., "Transplantation of Adult Human Corneal Endothelium Ex Vivo: A Morphologic Study," Cornea 20(7): 731-737, 2001. The tissue collected thus contains HCECs, but it may also contain corneal keratocytes (specialized fibroblasts residing the stroma). Corneal keratocytes (also referred to herein simply as "keratocytes") are undesirable contaminants in the HCECs culture, as they grow faster than the latter cells and they can take over the culture dish, thus making the final product essentially useless. In addition to residual stromal tissue, keratocytes may also arise from human endothelial cells which transform spontaneously into other types of cells such as keratocytes (see, e.g., G S. L. Peh et al., "Optimization of Human Corneal Endothelial Cells for Culture: The Removal of Corneal Stromal Fibroblast Contamination Using Magnetic Cell Separation," International Journal of Biomaterials, Volume 2012 (2012), Article ID 601302, 8 pages.). WO2005/038015A1 discloses a method for isolation and enrichment of human corneal endothelial cells and therapeutic uses thereof. "Stem cells of the adult cornea: From cytometric markers to therapeutic applications" by Lili Takács et al. 2009, Cytometry, Part A, vol. 75A no.1, Pages 54 to 66 reviews various cell types of the human adult cornea having stem cell-like characteristics and marker combinations used to identify and enrich those cells. EP3029140A1 discloses marker panels for the identification and enrichment of human corneal endothelial cells. "Novel Identity and Functional Markers for Human Corneal Endothelial Cells" by Alena Bartakova et al. 2016, Investigative Ophthalmology and Visual Science, vol. 57, no.6, page 2749 discloses a series of markers indicative of a shift in cultured human HCECs from canonical to fibroblastic morphology. WO2013/012087A1 discloses a method of preparing a corneal endothelial progenitor cellular culture. "CD marker expression profiles of human embryonic stem cells and their neural derivatives, determined using flow-cytometric analysis, reveal a novel CD marker for exclusion of pluripotent stem cells" by Sundberg M et al. 2009, vol. 2, no. 2 , pages 113 to 124 discloses that neural derivatives of hESCs have high levels of CD90, CD56 and CD166 markers.

### SUMMARY OF THE INVENTION

Some aspects of the invention are directed to methods for the identification, enrichment and/or isolation of human corneal endothelial cells (HCECs).

In some embodiments, two or more differing positive affinity reagents which bind to HCECs but which do not bind to cells other than HCECs are employed.

As defined herein "cells other than human corneal endothelial cells" (or "cells other than HCECs") include corneal keratocytes as well as HCECs of lower utility (e.g., HCECs that have undergone fibroblastic or mesenchymal transformation, etc.).

In some embodiments, the method comprises both (a) positive selection using one or more affinity reagents agents and (b) negative selection using one or more negative affinity reagents.

In accordance with an aspect of the present invention, there is provided a method of forming a composition enriched with human corneal endothelial cells as claimed in claim 1.

Furthermore, the first positive affinity reagent may be coupled to a label, wherein the second positive affinity reagent may be coupled to a label, and wherein the second affinity reagent may be coupled to a label.

In accordance with a further aspect of the present invention, there is provided a kit as claimed in claim 3.

In accordance with a further aspect of the present invention, there is provided a composition enriched with human corneal endothelial cells as claimed in claim 4.

These and various other aspects and embodiments and as well as various advantages of the present invention will become immediately apparent to those of ordinary skill in the art upon review of the Detailed Description and appended claims to follow.

### BRIEF DESCRIPTION OF THE DRAWINGS

Figs. 1A-1C are bright field micrographs of HCECs and keratocytes in culture.
Fig. 2 illustrates in bar graph form expression of four surface markers in different corneal cell populations analyzed by flow cytometry.
Figs. 3A-3C are dual-color fluorescence histograms of HCECs and keratocytes.
Fig. 4 presents Fluorescence profiles illustrating expression of four surface markers in in three different HCEC populations analyzed by flow cytometry.
Fig. 5 illustrates in bar graph form expression of four surface markers in three different HCEC populations analyzed by flow cytometry.
Fig. 6 illustrates dual-color fluorescence histograms for various pairs of surface markers in three different HCEC populations.
Fig. 7 illustrates trans-endothelial electrical resistance (TEER) as a function of time for cell cultures of three different HCEC populations.

### DETAILED DESCRIPTION

A more complete understanding of the present invention is available by reference to the following detailed description of numerous aspects and embodiments of the invention. The detailed description of the invention which follows is intended to illustrate but not limit the invention.

As noted above, in some aspects, the present disclosure, not in accordance with the present invention, pertains to positive selection processes in which cell populations containing human corneal cells are contacted with one or more positive affinity reagents that selectively bind to HCECs relative to cells other than HCECs (e.g., corneal keratocytes, etc.), including positive affinity reagents that selectively bind to HCECs that are likely to have a higher clinical efficacy relative to the general HCEC population.

In other aspects, not in accordance with the present invention, the disclosure pertains to negative selection processes in which cell populations containing human corneal cells are contacted with one or more negative affinity reagents that bind selectively bind to cells other than HCECs (e.g., corneal keratocytes, etc.) relative to HCECs.

These negative and positive selection methods may be used independently or in combination with one another, for example, to identifying HCECs, to isolate HCECs and/or to enrich cell populations with HCECs, among other uses.

Cell populations suitable for HCEC enrichment or isolation include those obtained from intact or residual human corneas, which may come, for instance, from embryonic, fetal, pediatric or adult tissue. For example, intact corneas may be subjected to a peel-off step in which the endothelium and its basement membrane (Descemet's membrane) are peeled off the stroma and collected. See Ko-Hua Chen et al., "Transplantation of Adult Human Corneal Endothelium Ex Vivo: A Morphologic Study," Cornea 20(7): 731-737, 2001. In other embodiments, cell populations may be obtained from residual corneas (e.g., eye tissue remaining after a corneal button has been used for DSAEK).

Tissue from intact and residual corneas may be separated into individual cells by processes such as enzymatic and/or mechanical dissociation. At this step, cells are incubated for a period of time at room temperature or at 37°C with a single enzyme or a combination of enzymes including some of the following: collagenase, papain, dispase, elastase, trypsin/EDTA, and/or DNAse. Later the tissues are mechanically dissociated using a conventional pipette or a glass pipette to obtain individual cells or cell clumps than can be then expanded in culture. See, e.g., Li W. et al., Invest Ophthalmol Vis Sci 2007; 48: 614; Ishino Y. et al., Invest Ophthalmol Vis Sci 2004; 45: 800; Chen K.H. et al., Cornea 2001; 20: 731.

The medium in which the cells may be suspended will be any medium which maintains the viability of HCECs. Various media are commercially available and may be used including Minimal Essential Medium (MEM), Dulbecco's Modified Eagle Medium (DMEM), Opti-MEM®, Media 199 or M199, Dulbecco's Modified Eagle Medium with Nutrient Mixture F-12 (DMEM/F-12), , F99 Ham's F12, SHEM Ham's F12, EGM-2 endothelial growth medium frequently supplemented with serum of human or animal origin, BSA, HSA, growth factors, antioxidants, antibiotics, antimicotic agents, hormones, amino acids, and peptides. Specific examples of media are shown in Table 1 to follow.

**Table 1.**

| **Base Medium** | **Serum** | **Growth Factors & Supplements** |
|---|---|---|
| [M1] | 10% | 2 ng/ml bFGF |
| DMEM | | 50 U/ml penicillin |
| | | 50 µg/ml streptomycin |
| [M2] | 8% | 20 ng/ml NGF |
| Opti-MEM-I | | 5 ng/ml EGF |
| | | 20 µg/ml ascorbic acid |
| | | 200 mg/L calcium chloride |
| | | 100 µg/ml pituitary extract |
| | | 50 µg/ml gentamicin |
| | | 1x antibiotic/antimycotic |
| | | 0.08% chondroitin sulphate |
| [M3] | 5% | 0.5% DMSO |
| SHEM Ham's F12 & DMEM (1:1 ratio) | | 2 ng/ml EGF |
| | | 5 µg/ml insulin |
| | | 5µg/ml transferrin |
| | | 5 ng/ml selenium |
| | | 0.5 µg/ml hydrocortisone |
| | | 1 nM cholera toxin |
| | | 50 µg/ml gentamicin |
| | | 1.25 µg/ml amphotericin B |
| [M4] | 5% | 20 µg/ml ascorbic acid |
| F99 Ham's F 12 & M100 (1:1 ratio) | | 20 µg/ml bovine insulin |
| | | 2.5 µg/mol transferrin |
| | | 0.6 ng/ml sodium selentite |
| | | 10 ng/ml bFGF |

Cell cultures from intact and residual corneas contain unwanted contaminant cells which arise from residual non-endothelial tissue (e.g., stroma, epithelium, etc.) that may be present in the sample. In a culture of HCECs, HCECs that are of low cell transplant utility compared to other HCECs of high cell transplant utility may also be considered, in some fashion, "contaminants".

Cell populations suitable for HCEC enrichment or isolation also include HCEC cultures in which contaminant cells have out-multiplied HCECs or in which HCECs have transformed spontaneously into other types of cells (e.g., keratocytes, etc.). As previously noted, contaminant cells such as keratocytes are particularly undesirable where it is desired to expand an HCEC culture ex vivo, because such cells grow faster than HCECs and can thus take over a cell culture.

Consequently, various aspects, not in accordance with the present invention, pertain to methods, reagents and kits for separation of HCECs from other cells, particularly, keratocytes and/or HCECs of lower utility. The HCECs are separated from mixtures of cells by techniques that select cells having particular characteristics.

Human corneal endothelial cells may identified or selected (a) through positive cell markers, which are cell markers that are found on the surfaces of HCECs but which are not found on the surfaces of contaminant cells which may be intermixed with HCECs (e.g., positive selection), (b) through negative cell markers, which are cell markers that are found on surfaces of contaminant cells that are intermixed with HCECs and but which are not found on the surfaces of HCECs (e.g., negative selection), and through a combination of positive and negative cell markers. In other words, human corneal endothelial cells may be identified or selected via a first positive affinity reagent and a second affinity reagent.

For example, in the case where whole human corneas are used as a source of endothelial cells, positive cell markers may be selected from corneal proteins which are found in the endothelium (which is formed from HCECs) but which are not found in other corneal tissue (i.e., the stroma and/or the epithelium). Conversely, negative cell markers may be selected from corneal proteins which are found in corneal tissue other than endothelium tissue (i.e., the stroma and/or the epithelium) but which are not found in corneal endothelium.

As another example, in the case where the source of endothelial cells is an endothelium and basement membrane that have been separated from the stroma and epithelium of an intact cornea, positive cell markers may be selected from corneal cell proteins which are found in the endothelium but which are not found in the stroma, while negative cell markers may be selected from corneal cell proteins which are found in the stroma but which are not found in corneal endothelium.

Corneal proteins which may be useful as cell markers in conjunction with the present invention include the suitable proteins selected from those presented in the Table 2 set forth in Appendix A.

Positive cell markers include suitable corneal proteins selected from protein products of genes X1-X26 in Table 2 (e.g., SEQ ID NO (1) through SEQ ID NO (58)) which are present in the corneal endothelium but are not present in the stroma or the epithelium.

Negative cell markers include (a) suitable corneal proteins selected from protein products of genes Y1-Y23 in Table 2 (e.g., SEQ ID NO (59) through SEQ ID NO (96)), which are present in the stroma and in epithelium but are not present in the endothelium and (b) suitable corneal proteins selected from protein products of genes Z1-Z8 in Table 2 (e.g., SEQ ID NO (97) through SEQ ID NO (109)), which are present in the stroma but are not present in the corneal endothelium (or epithelium).

As previously noted, in some aspects, not in accordance with the present invention, the disclosure pertains to (a) positive selection processes in which cell populations containing human corneal cells are contacted with one, two, three, four or more positive affinity reagents that selectively bind to HCECs relative to cells other than HCECs (e.g., corneal keratocytes, etc.), (b) negative selection processes in which cell populations containing human corneal cells are contacted with one, two, three, four or more negative affinity reagents that selectively bind to cells other than HCECs (e.g., corneal keratocytes, etc.) relative to HCECs, and (c) combinations of (a) and (b).

For this purpose, affinity reagents are employed which preferentially bind to various corneal proteins. Positive affinity reagents are those that preferentially bind to positive cell markers associated with HCECs while negative affinity reagents are those that preferably bind to negative cell markers associated with contaminant cells other than HCECs.

Various positive cell markers are described above and include corneal proteins which are found in the endothelium (which is formed from HCECs) but which are not found in other corneal tissue (i.e., the stroma and/or the epithelium). Various negative cell markers are also described above and include corneal proteins which are found in corneal tissue other than endothelium (i.e., the stroma and/or the epithelium) but which are not found in corneal endothelium.

Those skilled in the art will recognize that suitable negative and positive affinity reagents can be employed in any order and/or in any combination.

Affinity reagents suitable for use in the present disclosure may comprise any species which selectively binds to a given surface marker, including positive affinity reagents which selectively bind to positive cell markers and negative affinity reagents which selectively bind to negative cell markers.

Especially useful affinity reagents for the practice of the invention are antibodies (also referred to herein as "affinity antibodies"), nucleic acid aptamers and other engineered forms of protein scaffolds. Antibodies include whole antibodies and antibody fragments, e.g. Fab, F(ab')₂, light or heavy chain fragments, etc.

Affinity antibodies selected for use will have a low level of non-specific interactions.

Affinity antibodies may be polyclonal or monoclonal and, where not commercially available, may be readily produced by techniques known to those skilled in the art.

For instance, affinity antibodies to a given corneal protein may be obtained by immunizing a xenogeneic immunocompetent mammalian host (including murine, rodentia, lagomorpha, ovine, porcine, bovine, etc.) with the corneal protein of interest. Immunizations are performed in accordance with conventional techniques, where the corneal proteins may be injected subcutaneously, intramuscularly, intraperitoneally, intravascularly, etc., over a course of one or more injections. After completion of the immunization schedule, the antiserum may be harvested in accordance with conventional methods to provide polygonal antisera specific for the corneal protein of interest. Lymphocytes may also be harvested from the appropriate lymphoid tissue, e.g. spleen, draining lymph node, etc., and fused with an appropriate fusion partner, for example, a myeloma line, producing a hybridoma secreting a specific monoclonal antibody. Screening clones of hybridomas for the antigenic specificity of interest is performed in accordance with conventional methods.

In numerous embodiments, affinity antibodies are coupled to a suitable substrate, for example, a label or a solid matrix. Labels include magnetic labels such as magnetic beads or micro or nanoparticles including superparamagnetic nanoparticles, which allow for ease of separation. Labels also include biotin, which binds with high affinity to avidin or streptavidin. Labels further include fluorochromes, which can be used with flow cytometry, e.g., fluorescence activated cell sorting (FACS), or the like, to allow for ease of separation of a particular cell type. Fluorescence activated cell sorters have varying degrees of sophistication, such as multiple color channels, low angle and obtuse light scattering detecting channels, impedance channels, etc. Fluorochromes include phycobiliproteins, e.g., phycoerythrin and allophycocyanins, fluorescein and Texas red, cy7 and cy5, among others. Multiple antibodies each with an affinity to a particular corneal protein may each be labeled with a different fluorochrome, to permit independent sorting (multi-color analyses) for each associated cell protein.

Cell selection may also be achieved by "panning" with an affinity antibody attached to a solid matrix, e.g. a plate, an immobilized bead, and so forth. For example, an affinity antibody that has specificity for a particular corneal protein may be bound to a solid matrix and corneal cells displaying that particular corneal protein can be captured by the immobilized antibody while the other cells remain in suspension and can be removed.

Any sorting technique may be employed which is not unduly detrimental to the viability of the selected cells. Combinations of the above techniques may be used.

The precise method for coupling an antibody to a given substrate (e.g., a label, solid matrix, etc.) is not critical to the practice of the present disclosure, and a number of alternatives are known in the art. For example, affinity antibodies may directly or indirectly be coupled to a substrate. Direct coupling to a substrate can be achieved by use of various chemical linking groups, as known in the art. For example, an antibody can be coupled to a substrate through side chain amino or sulfhydryl groups and heterofunctional cross-linking reagents. Many heterofunctional compounds are available for linking to various entities. Specific examples include 3-(2-pyridyldithio)propionic acid N-hydroxysuccinimide ester (SPDP) or 4-(N-maleimidomethyl)-cyclohexane-1-carboxylic acid N-hydroxysuccinimide ester (SMCC), which can react with a reactive sulfhydryl group on the antibody and a reactive amino group on the substrate.

Alternatively, affinity antibodies can be indirectly coupled to a substrate via a hapten or a secondary antibody, not in accordance with the present invention. For instance, the antibody may be directly conjugated to a hapten, and hapten-specific binding species may be conjugated to the substrate. Suitable haptens include digoxin, digoxigenin, FITC, dinitrophenyl, nitrophenyl, avidin, streptavidin, biotin, etc. For example, an antibody may be coupled to one member of a high affinity binding system (e.g., biotin) and another member of the high affinity binding system (e.g., avidin) attached to a substrate. Methods for conjugation of a hapten to a protein are known in the art, and kits for such conjugations are commercially available. The secondary antibody may be directly or indirectly bound to the substrate.

During cell separation, coupled antibodies may be combined with a suspension of cells and incubated for a period of time sufficient for the antibodies to bind to proteins on the cells. The amount of antibody necessary to bind a particular cell subset may be empirically determined by performing a test separation and analysis. The cells and antibodies are incubated for a period of time sufficient for binding to occur.

The medium in which the cells are separated will be any medium which maintains the viability of the cells. Various media are commercially available and include those listed above.

Coupled affinity antibodies include coupled positive affinity antibodies specific for the corneal proteins which are present on human corneal endothelial cells and which are not present on contaminant cells such as stromal and/or epithelial cells (for positive selection) and coupled negative affinity antibodies specific for corneal proteins which are present on contaminant cells such as stromal and/or epithelial cells and which are not present on human corneal endothelial cells (negative selection).

Once the antibody is bound to the cell, the bound cells are separated in accordance with the specific antibody preparation. For example, FACS separation may be used with fluorochrome labeled antibodies, immunomagnetic selection may be used with magnetic-labeled antibodies, "panning" may be employed with immobilized antibodies, and so forth.

Cells may be separated from affinity antibodies using known techniques, as desired. As a specific example, where an antibody in an immunopanning process is a positive selection antibody, the matrix with attached endothelial cells may be washed to remove unbound cells and the endothelial cells released using a suitable technique (e.g., trypsin digest).

While various specific embodiments employing antibodies as affinity reagents are specifically described herein, it is to be understood that other affinity reagents for binding positive or negative cell markers can be used in the same fashion, including nucleic acid aptamers and other engineered forms of protein scaffolds. Aptamers are synthetic oligonucleotides selected from pools of random-sequence oligonucleotides which bind to a wide range of biomolecular targets with high affinity and specificity. See, e.g., J. Wang and G. Li, "Aptamers against cell surface receptors: selection, modification and application," Curr Med Chem. 2011;18(27):4107-16.

The separated cells may be collected in any appropriate medium that maintains the viability of the cells.

Cell populations enriched with HCECs may thus be achieved in this manner. The HCEC population may constitute 50% or more of the cells in the cell composition, preferably at 75% or more of the cells in the cell composition, more preferably at 90% or more of the cells in the cell composition, and may be as many as 95% or more (e.g. substantially pure) of the cells in the cell population. Conversely, the cell populations may contain up to 50% of cells other than HCECs (e.g., corneal keratocytes, etc.), for instance 50% or less of such cells, preferably 25% or less of such cells, more preferably 10% or less of such cells, and may be as few as 5% or less of such cells.

The enriched cell population may be used immediately or stored. For example, at room temperature, at 4°C, at 37°C or the cells may be frozen at liquid nitrogen temperatures and stored for long periods of time.

In certain embodiments, not in accordance with the present invention, the enriched cells may be further expanded in vitro by adding culture media as described widely in the literature. See, e.g., Li W et al., Invest Ophthalmol Vis Sci 2007; 48: 614. ; Ishino Y et al., Invest Ophthalmol Vis Sci 2004; 45: 800; Chen KH et al., Cornea 2001; 20: 731.

The enriched HCEC compositions thus obtained have a variety of uses in clinical therapy, research, development, and commercial purposes.

For example, for therapeutic purposes, human corneal endothelial cells may be ocularly administered to an eye of a patient in order to treat corneal endothelial cell loss or dysfunction.

Other aspects, not in accordance with the present invention, pertain to kits for conducting cell separations as described herein. Such kits may include any combination of the following, among other elements: (a) one, two, three or more positive affinity reagents, each of which may be, for example, in the form of a positive affinity antibody attached to a suitable substrate such as a solid matrix (e.g. a plate, immobilized bead, etc.) or label (e.g., magnetic label, fluorescent label, etc.), (b) one, two, three or more unlabeled positive affinity antibodies, which the end user could label using standard methods, choosing their preferred labels (e.g., fluorophores, haptens, etc.), (c) one, two, three or more negative affinity reagents, each of which may be, for example, in the form of a negative affinity antibody attached to a suitable substrate such as a solid matrix (e.g. a plate, immobilized bead, etc.) or label (e.g., magnetic label, fluorescent label, etc.), (d) or one, two, three or more unlabeled negative affinity antibodies, which the end user could label using standard methods, choosing their preferred labels (e.g., fluorophores, haptens, etc.); (e) a combination of (a) and (c); (f) a combination of (b) and (d); (g) packaging; (h) printed materials with one or more of the following: (i) storage information and (ii) instructions regarding how to use the materials contained in the kit (e.g., positive affinity reagents, negative affinity reagents, a combination of antibodies for sequential use, etc.).

### EXAMPLE 1

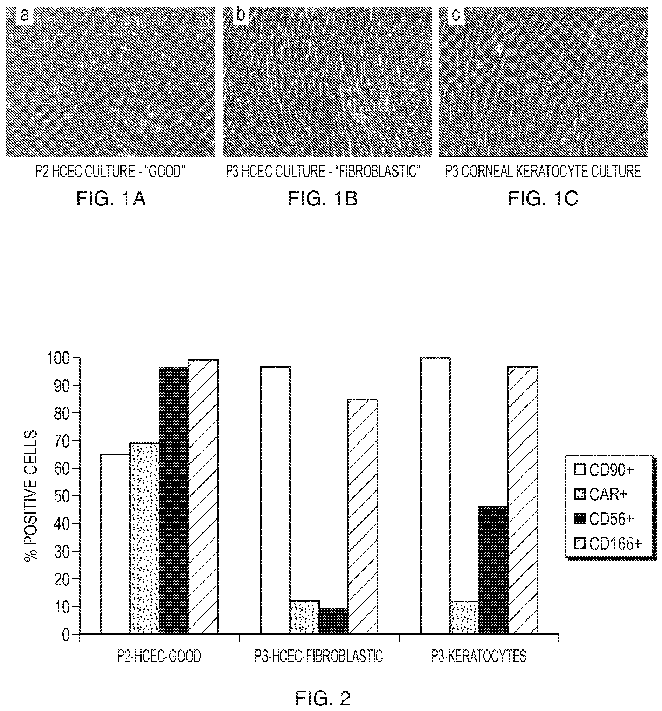

HCECs were isolated from cadaveric donor corneas (Tampa Lions Eye Bank) and cultured and expanded following the method described by Joyce and Zhu in Cornea. 2004 Nov;23(8 Suppl):S8-S19. Briefly, the endothelium and Descemet's membrane were peeled off of the stroma and after overnight stabilization at 37°C in Opti-MEM® media (Gibco, Life Technologies Corp, Carlsbad, CA), supplemented with 8% fetal bovine serum (FBS), they were incubated for 1hr at 37°C with ethylenediaminetetraacetic acid (EDTA) to loosen up the cell-cell interactions. Cells were then mechanically dissociated to obtain a single-cell suspension, they were seeded onto FNC-coated culture wells and labeled as "P0" (passage zero). After reaching confluency, they were trypsinized and further expanded into more wells to increase their number. After one or two rounds of expansion, cells were collected and incubated with different antibodies as indicated below. Keratocytes were also obtained from cadaveric donor corneas using the method described by Stramer et al. in "Monoclonal antibody (3G5)-defined ganglioside: cell surface marker of corneal keratocytes," Invest. Ophthalmol. Vis. Sci. 2004 vol. 45 no. 3 807-812. While one of the HCEC cultures preserved its typical cobblestone morphology at passage 2 (Fig. 1A), a second culture underwent endothelial-to-mesenchymal transition during passage 3 (P3) and the cells became fibroblastic (Fig. 1B). Such cells are generally referred to herein as human corneal endothelial cells of lower utility (e.g., HCECs that have undergone fibroblastic or mesenchymal transformation, etc.). The keratocyte culture, not in accordance with the present invention, exhibits the typical fibroblastic, elongated cell morphology (Fig. 1C).

HCECs from each culture and keratocytes, not in accordance with the present invention, were collected and incubated with one or more of the following labelled antibodies: (a) APC-CD56 which is a mouse monoclonal antibody against a protein product of gene X15 from Table 2 (referred herein to as CD56 surface protein) coupled to allophycocyanin (BD Biosciences, #555518), (b) PE-CD166, not in accordance with the present invention, which is a mouse monoclonal antibody against a protein product of gene X1 from Table 2 (referred here to as CD166 surface protein) coupled to phycoerythin (BD Biosciences #559263), (c) FITC-CAR, which is a mouse monoclonal antibody against a protein product of gene X25 from Table 2 (referred to as CAR surface protein) coupled to fluorescein-5-Isothiocyanate (Santa Cruz Biotechnology, Santa Cruz, California, USA #sc-56892) and (d) PECy7-CD90, not in accordance with the present invention, which is a mouse monoclonal antibody against a protein product of gene Z8 from Table 2 (referred to as CD90 surface protein) coupled to a tandem conjugate of PE (energy donor) which has an excitation wavelength of 565nm and Cy7 (energy acceptor) which has an emission wavelength of 778nm) (BD Biosciences #561558).

Expression of surface markers was analyzed using a BD LSR™II flow cytometry system (BD Biosciences, San Jose, CA). The data shown in Fig 2 are representative from one experiment. Similar results were obtained upon repeated experimentation. Quantification of the % positive cells for each marker shows that in fibroblastic cultures there is a decreased expression of CD56 and CAR, indicating that antibodies to these proteins may be used in conjunction with positive affinity reagents for "good" HCECs. A significant difference in the expression of CD166 or CD90 was not detected using this particular antibody.

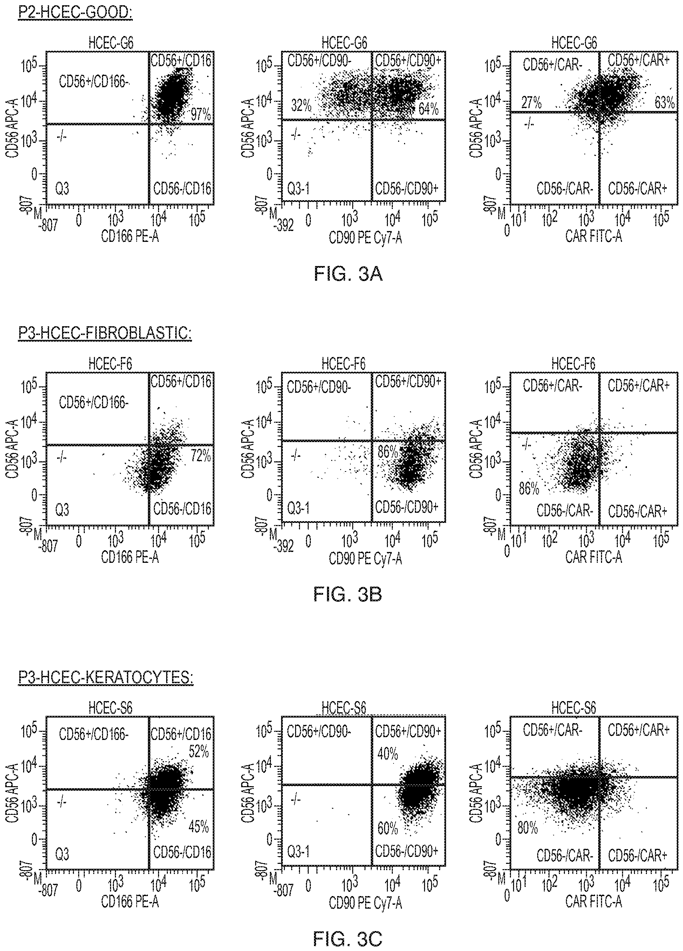

Figs. 3A-3C are dual-color fluorescence dot plots of the HCECs and keratocytes. These dot plots show the differential expression of two surface markers in each cell population as labeled. The percent of cells positive for an individual marker is shown in Fig 2.

### EXAMPLE 2

HCECs were isolated from cadaveric donor corneas as described in Example 1. Also as discussed in Example 1, HCEC cultures were obtained (a) which evidenced a typical cobblestone morphology (referred to in this Example 2 as a "canonical" cell culture), (b) where all the cells had undergone an endothelial-to-mesenchymal transition (referred to in this Example as a "fibroblastic" cell culture) and (c) where some HCECs had undergone endothelial-to-mesenchymal transition (referred to in this Example as a "mixed" cell culture).

HCEC surface markers were identified by microarray data, and several with high expression in the endothelium (cultured and freshly dissected) but low expression in stroma were selected to be tested by flow cytometry analysis. In addition to APC-CD56, PE-CD166 not in accordance with the present invention, FITC-CAR and PECy7-CD90 not in accordance with the present invention, described in Example 1, also tested were (e) CD109-PE, (i.e., mouse anti-CD109), which is a monoclonal antibody against a protein product of gene Y6 from Table 2 (referred to as CD109 antigen) conjugated to phycoerythrin (PE), BD Biosciences Cat# 556040 and (f) CD 248-BV, (i.e., mouse anti-Endosialin), which is an unconjugated monoclonal antibody against a protein product of gene X5 from Table 2 (referred to as CD248 antigen or Endosialin), (Millipore, Temecula, CA, USA, Cat# MAB2626), incubated with Goat polyclonal anti-Mouse IgG secondary antibody conjugated to Brilliant Violent 421 (Biolegend, Inc., San Diego, CA, USA, Cat# 405317).

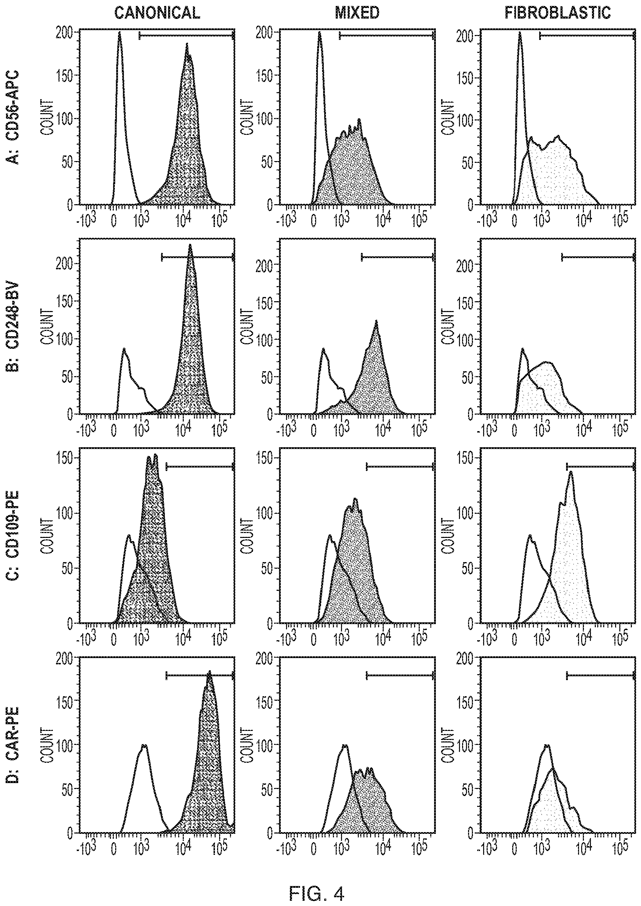

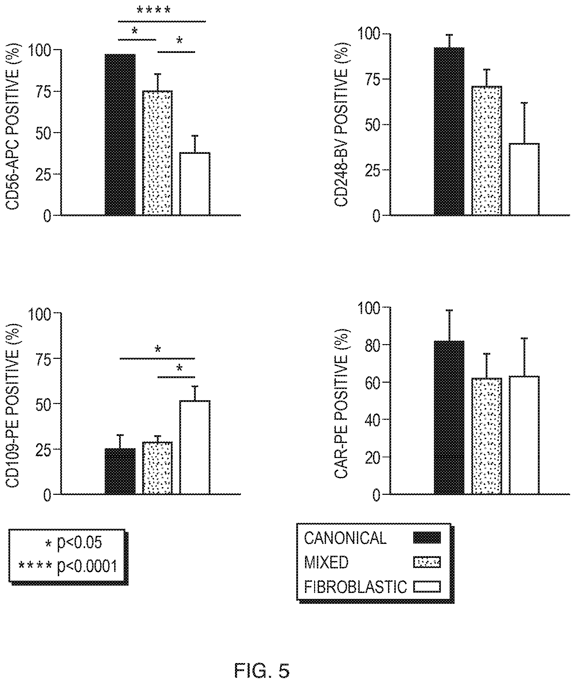

To address whether the expression of those markers in HCECs were affected by the fibroblastic conversion described above, HCEC cultures demonstrating two different morphologies (canonical and fibroblastic) and a corneal keratocyte culture as a control were immunostained for the surface proteins CD90 not in accordance with the present invention, CAR, CD56 and CD166 (See Example 1, Fig. 2). CD56, CAR, CD109 and CD248 expression was also compared between canonical (good), mixed, and fibroblastic HCECs (see Figs. 4 and 5). Analysis of the percentage of cells expressing any of the individual markers in canonical and fibroblastic cultures demonstrated that CD56, CAR and CD248 expression was reduced in the fibroblastic culture (see Fig. 5), while CD109 was elevated (see Fig. 5); CD90 not in accordance with the present invention, and CD166 expression did not significantly change between good/canonical and fibroblastic cultures (see Example 1, Fig. 2). A comparable trend was observed in the keratocyte culture used as control for CD90, CAR, CD56 and CD166 expression (see Example 1, Fig. 2).

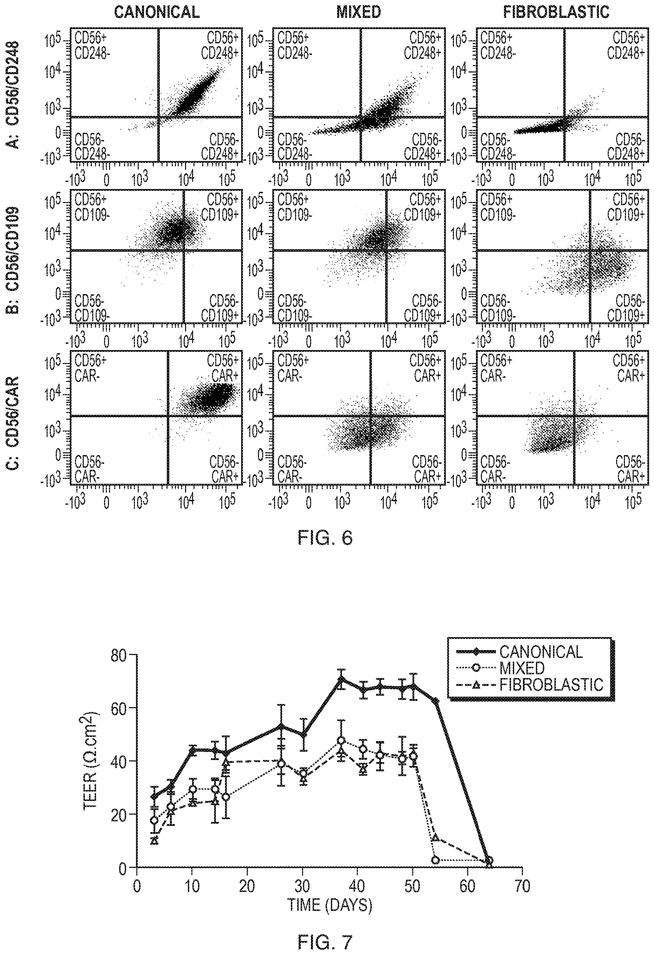

Dot plot dual histograms of canonical, mixed and fibroblastic cultures shown in Fig. 6 demonstrated that canonical HCECs are predominantly CD56, CD248 and CAR positive, and CD109 negative; CD56 and CD248 expression is lost and CD109 expression increases as the culture becomes fibroblastic.

Finally, trans-endothelial electrical resistance (TEER) of cell cultures was measured. HCECs (a) from "good" or "canonical" cultures that expressed high levels of CD56, (b) from mixed cultures and (c) from fibroblastic cultures were plated onto inserts with 0.4 mm pores in 24-well culture plates (Transwell, Corning Costar, Acton, MA) at a density of 20,000 cells/insert and incubated in growth media as described in Example 1. TEER was measured using an EVOM volt-ohm meter with STX2 Electrode (World Precision Instrument, Inc., Sarasota, FL) for up to 65 days after initial plating. TEER measures the apical and basal plasma membrane resistance and the paracellular resistance and is used as an index of monolayer confluence integrity of tight junctions. To calculate final resistance (Ω·cm2), the resistance of blank filters were subtracted from those of filters with cells. Four wells per condition were averaged. HCECs exhibiting a canonical morphology and being CD56-positive demonstrated a superior barrier formation ability measured by TEER (Fig. 7).

Thus, we have identified a panel of surface makers that can be used to characterize a canonical and functionally superior HCEC culture, and may be used as quality control criteria or to potentially separate the best HCEC subpopulations for expansion.

## Claims

1. A method of forming a composition enriched with human corneal endothelial cells comprising: contacting a cell population containing human corneal cells with a first positive affinity reagent that selectively binds to human corneal endothelial cells relative to human corneal endothelial cells that have undergone a fibroblastic transformation and selecting cells to which the first positive affinity reagent is bound, wherein said first positive affinity reagent comprises a CD56 antibody; and further comprising contacting said cell population containing human corneal cells with a further affinity reagent, wherein the further affinity reagent is any one of: a second positive affinity reagent comprising a CD248 antibody; a second positive affinity reagent comprising a coxsackie virus and adenovirus receptor antibody; or a second affinity reagent comprising a CD109 antibody.

2. The method of claim 1, wherein the first positive affinity reagent is coupled to a label, wherein the second positive affinity reagent is coupled to a label, and wherein the second affinity reagent is coupled to a label.

3. A kit comprising (a) a positive affinity reagent that selectively binds to human corneal endothelial cells relative to human corneal endothelial cells that have undergone a fibroblastic transformation, wherein said positive affinity reagent comprises a CD56 antibody and (b) a further affinity reagent, wherein the further affinity reagent is any one of: a second positive affinity reagent comprising a CD248 antibody; a second positive affinity reagent comprising a coxsackie virus and adenovirus receptor antibody; or a second affinity reagent comprising a CD109 antibody.

4. A composition enriched with human corneal endothelial cells comprising: (a) human corneal cells; (b) a first positive affinity reagent that selectively binds to human corneal endothelial cells relative to human corneal endothelial cells that have undergone a fibroblastic transformation, wherein said first positive affinity reagent comprises a CD56 antibody; and (c) a further affinity reagent, wherein the further affinity reagent is any one of: a second positive affinity reagent comprising a CD248 antibody; a second positive affinity reagent comprising a coxsackie virus and adenovirus receptor antibody; or a second affinity reagent comprising a CD109 antibody.

## Patentansprüche

1. Verfahren zur Bildung einer Zusammensetzung, die mit menschlichen Hornhautendothelzellen angereichert ist, umfassend: Inkontaktbringen einer Zellpopulation, die humane Hornhautzellen enthält, mit einem ersten positiven Affinitätsreagenz, das selektiv an humane Hornhautendothelzellen im Vergleich zu humanen Hornhautendothelzellen bindet, die eine fibroblastische Transformation durchlaufen haben und Auswählen von Zellen, an die das erste positive Affinitätsreagenz gebunden ist, wobei das erste positive Affinitätsreagenz einen CD56-Antikörper umfasst; und ferner umfassend das Inkontaktbringen der Zellpopulation, die menschliche Hornhautzellen enthält, mit einem weiteren Affinitätsreagenz, wobei das weitere Affinitätsreagenz eines der folgenden ist: ein zweites positives Affinitätsreagenz, umfassend einen CD248-Antikörper; ein zweites positives Affinitätsreagenz, umfassend einen Coxsackie-Virus- und Adenovirus-Rezeptor-Antikörper; oder ein zweites Affinitätsreagenz, umfassend einen CD109-Antikörper.

2. Verfahren nach Anspruch 1, wobei das erste positive Affinitätsreagenz an eine Markierung gekoppelt ist, wobei das zweite positive Affinitätsreagenz an eine Markierung gekoppelt ist und wobei das zweite Affinitätsreagenz an eine Markierung gekoppelt ist.

3. Kit, umfassend (a) ein positives Affinitätsreagenz, das selektiv an menschliche Hornhautendothelzellen im Vergleich zu menschlichen Hornhautendothelzellen bindet, die eine fibroblastische Transformation durchlaufen haben, wobei das positive Affinitätsreagenz einen CD56-Antikörper umfasst, und (b) ein weiteres Affinitätsreagenz, wobei das weitere Affinitätsreagenz eines der folgenden ist: ein zweites positives Affinitätsreagenz, umfassend einen CD248-Antikörper; ein zweites positives Affinitätsreagenz, umfassend einen Coxsackie-Virus- und Adenovirus-Rezeptor-Antikörper; oder ein zweites Affinitätsreagenz, umfassend einen CD109-Antikörper.

4. Mit menschlichen Hornhautendothelzellen angereicherte Zusammensetzung, umfassend:
(a) menschliche Hornhautzellen; (b) ein erstes positives Affinitätsreagenz, das selektiv an humane Hornhautendothelzellen im Vergleich zu humanen Hornhautendothelzellen bindet, die eine fibroblastische Transformation durchlaufen haben, wobei das erste positive Affinitätsreagenz einen CD56-Antikörper umfasst; und (c) ein weiteres Affinitätsreagenz, wobei das weitere Affinitätsreagenz eines der folgenden ist: ein zweites positives Affinitätsreagenz, umfassend einen CD248-Antikörper; ein zweites positives Affinitätsreagenz, umfassend einen Coxsackie-Virus- und Adenovirus-Rezeptor-Antikörper; oder ein zweites Affinitätsreagenz, umfassend einen CD109-Antikörper.

## Revendications

1. Procédé de formation d'une composition enrichie en cellules endothéliales de la cornée humaine comprenant : la mise en contact d'une population cellulaire contenant des cellules de la cornée humaine avec un premier réactif d'affinité positive qui se lie sélectivement aux cellules endothéliales de la cornée humaine par rapport aux cellules endothéliales de la cornée humaine qui ont subi une transformation fibroblastique et la sélection des cellules auxquelles le premier réactif d'affinité positive est lié, dans lequel ledit premier réactif d'affinité positive comprend un anticorps CD56 ; et comprenant en outre la mise en contact de ladite population cellulaire contenant des cellules de la cornée humaine avec un réactif d'affinité supplémentaire, dans lequel le réactif d'affinité supplémentaire est l'un quelconque parmi : un second réactif d'affinité positive comprenant un anticorps CD248 ; un second réactif d'affinité positive comprenant un anticorps dirigé contre le récepteur des virus Coxsackie et des adénovirus ; ou un second réactif d'affinité comprenant un anticorps CD109.

2. Procédé selon la revendication 1, dans lequel le premier réactif d'affinité positive est couplé à un marqueur, dans lequel le second réactif d'affinité positive est couplé à un marqueur, et dans lequel le second réactif d'affinité est couplé à un marqueur.

3. Kit comprenant (a) un réactif d'affinité positive qui se lie sélectivement aux cellules endothéliales de la cornée humaine par rapport aux cellules endothéliales de la cornée humaine qui ont subi une transformation fibroblastique, dans lequel ledit réactif d'affinité positive comprend un anticorps CD56 et (b) un réactif d'affinité supplémentaire, dans lequel le réactif d'affinité supplémentaire est l'un quelconque parmi : un second réactif d'affinité positive comprenant un anticorps CD248 ; un second réactif d'affinité positive comprenant un anticorps dirigé contre le récepteur des virus Coxsackie et des adénovirus ; ou un second réactif d'affinité comprenant un anticorps CD109.

4. Composition enrichie en cellules endothéliales de la cornée humaine comprenant : (a) des cellules de la cornée humaine ; (b) un premier réactif d'affinité positive qui se lie sélectivement aux cellules endothéliales de la cornée humaine par rapport aux cellules endothéliales de la cornée humaine qui ont subi une transformation fibroblastique, dans lequel ledit premier réactif d'affinité positive comprend un anticorps CD56 ; et (c) un réactif d'affinité supplémentaire, dans lequel le réactif d'affinité supplémentaire est l'un quelconque parmi : un second réactif d'affinité positive comprenant un anticorps CD248 ; un second réactif d'affinité positive comprenant un anticorps dirigé contre le récepteur des virus Coxsackie et des adénovirus ; ou un second réactif d'affinité comprenant un anticorps CD109.
